# EUROPÄISCHE PATENTSCHRIFT

(11) **EP 1 499 896 B1**
(45) Veröffentlichungstag und Bekanntmachung des Hinweises auf die Patenterteilung: **23.08.2006**
(21) Anmeldenummer: 03724960.4
(22) Anmeldetag: 04.04.2003
(51) Int. Cl.: G01N 33/574, G01N 33/564

(54) **VERFAREN ZUR DIAGNOSE VON NEOPLASMEN**
METHOD FOR DIAGNOSING NEOPLASMS
METHODE POUR DIAGNOSTIQUER DES NEOPLASMES

(30) Priorität: 02.05.2002 EP 02009884
(43) Veröffentlichungstag der Anmeldung: 26.01.2005
(73) Patentinhaber: B.R.A.H.M.S Aktiengesellschaft, 16761 Hennigsdorf (DE)
(72) Erfinder: BERGMANN, Andreas, 12351 Berlin (DE)
(74) Vertreter: Andrae, Steffen
(86) Internationale Anmeldenummer: PCT/EP2003/003552
(87) Internationale Veröffentlichungsnummer: WO 2003/093825

(56) Entgegenhaltungen:
- KONSTANDOULAKIS M M ET AL: "AUTOANTIBODIES IN THE SERUM OF PATIENTS WITH GASTRIC CANCER: THEIR PROGNOSTIC IMPORTANCE" HYBRIDOMA, LIEBERT, NEW YORK, NY, US, Bd. 17, Nr. 5, Oktober 1998 (1998-10), Seiten 431-435, XP008008268 ISSN: 0272-457X in der Anmeldung erwähnt
- LEWARTOWSKA ALEKSANDRA ET AL: "Ganglioside reactive antibodies of IgG and IgM class in sera of patients with differentiated thyroid cancer." IMMUNOLOGY LETTERS, Bd. 80, Nr. 2, 1. Februar 2002 (2002-02-01), Seiten 129-132, XP001113115 ISSN: 0165-2478 in der Anmeldung erwähnt
- ESCANDE-BEILLARD NATHALIE ET AL: "A sensitive flow cytometry method for anti-GM1 antibodies detection." JOURNAL OF NEUROIMMUNOLOGY. NETHERLANDS APR 2002, Bd. 125, Nr. 1-2, April 2002 (2002-04), Seiten 163-169, XP001113027 ISSN: 0165-5728
- BECH E ET AL: "ELISA-type titertray assay of IgM anti-GM1 autoantibodies." CLINICAL CHEMISTRY. UNITED STATES JUL 1994, Bd. 40, Nr. 7 Pt 1, Juli 1994 (1994-07), Seiten 1331-1334, XP001108903 ISSN: 0009-9147

## Beschreibung

Die Erfindung betrifft ein Verfahren zur Diagnose von Neoplasmen bei einem menschlichen Patienten, insbesondere zu deren Früherkennung, sowie zur Abschätzung der Gefährdung eines menschlichen Patienten durch eine Ausbildung und/oder Ausbreitung maligner Neoplasmen, indem man in einer biologischen Probe eine "universelle Krebs-Markersubstanz" bestimmt.

Die erfindungsgemäß bestimmte Markersubstanz ist eine humorale Markersubstanz, die mit sehr hoher Sensitivität (richtiger Erkennung von Proben von Kranken) das Vorhandensein von Neoplasmen bei Patienten anzeigt und darüber hinaus zusätzlich auch eine besondere Gefährdungslage/Prognose für Patienten, bei denen sich derartige Neoplasmen noch nicht klinisch manifestieren, die jedoch aufgrund z.B. ihrer Lebensumstände (z.B. einer beruflichen Exposition; Rauchen) und/oder einer Vorerkrankung und/oder medizinischen Behandlung einem besonderen statistischen Risiko ausgesetzt sind, eine maligne neoplastische Erkrankung zu entwickeln.

Der Begriff "Neoplasma" wird im Rahmen der vorliegenden Anmeldung als Oberbegriff bzw. Synonym für Tumoren, insbesondere bösartige Tumoren, verwendet, wie sie für Krebserkrankungen (Karzinome) typisch sind. An bösartigen Neubildungen (malignen Neoplasmen), d.h. Krebs, sind in einem Land wie Deutschland pro Jahr mehr als 300.000 Männer und Frauen erkrankt, wobei die Zahl der jährlich diagnostizierten neuen Krebsfälle sowie die Sterblichkeit erheblich sind (vgl. (1); in der nachfolgenden Beschreibung werden generell Querverweise auf Quellen als in Klammern gesetzte Zahlen angegeben; die Zahlen beziehen sich auf die Literaturliste am Ende der Beschreibung). Maligne Neoplasmen können in nahezu jedem Gewebe bzw. Organ entstehen, und je nach betroffenem Organ unterscheidet man zahlreiche Krebserkrankungen, die sich im Hinblick auf ihre statistische Häufigkeit, Prognose und Therapierbarkeit erheblich voneinander unterscheiden können.

In den letzten Jahren wurde eine Vielzahl von Therapien entwickelt, die im Hinblick auf die Heilbarkeit zahlreicher Formen von Krebserkrankungen erhebliche Fortschritte mit sich gebracht haben. In allen Fällen gilt jedoch, dass die Heilungschancen von Krebserkrankungen stets besser werden, je früher die Krebserkrankung erkannt wird. Ganz besonders wichtig ist dabei eine Früherkennung von Krebs, d.h. eine Erkennung von Neoplasmen, zu einem Zeitpunkt, zu dem noch keine oder noch keine signifikanten klinischen Symptome auftreten.

Zur möglichst frühen Erkennung von Neoplasmen in der klinischen Diagnose werden in biologischen Proben, insbesondere Blutproben und Körpersekreten, von untersuchten Patienten sog. "Tumormarker" bestimmt. Tumormarker sind Substanzen, die entweder von malignen Tumorzellen direkt gebildet werden oder die dadurch entstehen, dass Tumorzellen die Synthese des jeweiligen Markers in Nicht-Tumorzellen induzieren. Werden Tumormarker in erhöhter Konzentration in flüssigen biologischen Proben (humorale Tumormarker) bzw. im Gewebe lokalisiert (zelluläre Tumormarker), ermöglichen sie Schlüsse auf das Vorliegen, den Verlauf und die Prognose einer Tumorerkrankung. Die derzeit in der klinischen diagnostischen Praxis eingeführten Tumormarker können onkofetale Antigene, mit monoklonalen Antikörpern erkennbare Kohlenhydratepitope, Enzyme, Isoenzyme, onkogene Produkte und Rezeptoren sein. Ein Überblick über die derzeit in der klinischen Diagnostik genutzten Tumormarker findet sich in (2).

Allen derzeit bestimmten Tumormarkern ist gemeinsam, dass sie einerseits eine relativ hohe Organspezifität aufweisen, dass die Sensitivität ihrer Bestimmung jedoch gleichzeitig relativ beschränkt ist. Die relativ hohe Organspezifität der gegenwärtig bestimmten Tumormarker hat einerseits den Vorteil, dass man bei ihrem Nachweis gleichzeitig eine Information über das Organ erhält, bei dem die ursächliche Krebserkrankung mit hoher Wahrscheinlichkeit aufgetreten ist. Die hohe Spezifität ist jedoch insofern ein Nachteil, als Krebserkrankungen anderer Organe bei der Bestimmung organspezifischer Tumormarker nicht erkennbar werden bzw. für eine umfassende Krebsfrüherkennung die gleichzeitige Bestimmung zahlreicher verschiedener Tumormarker erforderlich ist. Die relativ geringe Sensivität der Bestimmung der bekannten Tumormarker (bei einer hohen Sensitivität einer Bestimmung werden die meisten oder alle Erkrankten richtig erkannt), die je nach Krebserkrankung und Tumormarker zwischen 20 und 80% liegt, hat zur Folge, dass die Gefahr einer Nichterkennung von Krebserkrankungen trotz der Bestimmung der an sich dafür geeigneten Tumormarker immer noch recht hoch ist.

Angesichts der skizzierten Situation wäre es außerordentlich wünschenswert, ein Verfahren zur Hand zu haben, das mit einer sehr hohen Sensitivität zuverlässige Hinweise auf das Vorliegen irgendeiner Tumorerkrankung (d.h. maligner Neoplasmen) liefert, so dass dann, ggf. unter zusätzlicher Berücksichtigung des klinischen Bilds des untersuchten, Patienten zur Vermeidung von Fehlinterpretationen der Messergebnisse, ggf. weitere diagnostische Maßnahmen zur genaueren Lokalisierung und differenzialdiagnostischen Bestimmung der nachgewiesenen neoplastischen Erkrankung gerechtfertigt sind. Gleichzeitig sollten bei einem solchen Verfahren Gesunde im Normalfall als solche erkennbar sein, so dass ein diagnostischer Fehlalarm möglichst vermieden werden kann. Derzeit existiert noch kein Verfahren, das die geschilderten Anforderung erfüllt, und angesichts der hohen Spezifitäts- und Sensivitätsunterschiede der bisher bekannten Tumormarker bestand auch wenig Anlaß zu der Hoffnung, dass ein universeller, hochsensitiver Tumormarker mit einer geringen Organspezifität überhaupt existiert und für die klinische Diagnostik nutzbar gemacht werden kann.

Die vorliegende Erfindung beruht auf der außerordentlich überraschenden Feststellung, dass ein bestimmter, an sich aus anderen Zusammenhängen bekannter Antikörper bzw. Autoantikörper, soweit derzeit experimentell überprüfbar war, bei allen getesteten malignen Neoplasmen (Krebsarten) in biologischen Proben, insbesondere Patientenseren, diagnostisch signifikant erhöht gefunden wird, während der gleiche Antikörper bei gesunden Normalpersonen nicht oder nur in deutlich geringeren Mengen nachweisbar ist. Durch die Bestimmung dieses Antikörpers wird es gemäß der vorliegenden Erfindung möglich, bei Patienten oder Personen, die sich einer Routineuntersuchung unterziehen oder an einer Reihenuntersuchung teilnehmen, mit hoher Zuverlässigkeit das Vorliegen maligner Neoplasmen zu erkennen, ohne dass eine signifikante Anzahl von gesunden Normalpersonen falsch positiv ermittelt wird. Soweit der bestimmte Antikörper auch bei einigen speziellen anderen Erkrankungen in erhöhten Konzentrationen auftritt, ist in der Regel eine korrekte Interpretation der Meßergebnisse aufgrund zusätzlicher klinischer Befunde ohne größere Schwierigkeiten möglich.

Gemäß der vorliegenden Erfindung wird somit gemäß Anspruch 1 ein *in vitro* Verfahren zur Früherkennung von Neoplasmen bei einem Patienten und zur Abschätzung seiner Gefährdung durch eine Ausbildung und/oder Ausbreitung maligner Neoplasmen geschaffen, bei dem man in einer Körperflüssigkeit des Patienten die Anwesenheit und Menge von an Asialo-G_{M1} (AG_{M1}) bindenden Antikörpern (anti-AG_{M1}-Antikörper) vom IgG- und/oder IgA-Typ bestimmt und deren Anwesenheit und/oder gegenüber Normalpersonen signifikant erhöhte Mengen mit dem Vorliegen eines Neoplasmas und/oder einer Gefährdung des Patienten durch maligne Neoplasmen korreliert.

Vorteilhafte Varianten und Ausgestaltungen des erfindungsgemäßen Verfahrens werden durch die nachfolgenden Ansprüche 2 bis 8 hervorgehoben.

Ein besonders wichtiger Aspekt der vorliegenden Erfindung kann als *in vitro* Verwendung von an AG_{M1} bindenden Autoantikörpern vom IgG- und/oder IgA-Typ als universelle Marker für die Früherkennung und Prognose von malignen neoplastischen Erkrankungen und für die Abschätzung des Risikos eines Patienten, dass sich bei ihm eine maligne neoplastische Erkrankung entwickelt, zusammengefaßt werden.

Nachfolgend werden die Auffindung des erfindungsgemäßen Diagnoseverfahrens unter Präsentation der diesem Verfahren zugrundeliegenden Meßwerte sowie eine derzeit bevorzugte Art seiner praktischen Durchführung noch in näheren Einzelheiten erläutert. Anschließend werden eine Deutung und ex-post-Plausibilisierung des erfindungsgemäßen Verfahrens im Lichte wissenschaftlicher Veröffentlichungen, die mit dem Gegenstand der vorliegenden Erfindung in einen Zusammenhang gebracht werden können, gegeben, die zeigen, dass die erfindungsgemäße Bestimmung von anti-Gangliosid-Antikörpern bzw. Autoantikörpern, insbesondere von solchen der genannten Art, auch für eine neue Deutung der Krebsentstehung bzw. -entwicklung hoch bedeutsam ist, wobei die der vorliegenden Erfindung zugrunde liegenden Befunde klare Hinweise auf neue therapeutische Ansätze zur Krebsprävention, Krebshemmung sowie ggf. Krebstherapie liefern.

Die vorliegende Erfindung ist ein Ergebnis der intensiven Forschungen der Anmelderin auf dem Gebiet der klinischen Diagnose von Autoimmunerkrankungen. Sie geht aus von der bekannten Erkenntnis, dass zu den Antikörpern, die in der Literatur im Zusammenhang mit Autoimmunerkrankungen, insbesondere nervenschädigenden, neuropathischen Autoimmunerkrankungen, diskutiert werden, auch bestimmte Anti-Gangliosid-Antikörper gehören.

Ganglioside sind Glykolipide, die Bestandteile der extrazellulären Seite der Plasmamembran tierischer Zellen sind und als solche auch im Nervengewebe auftreten. Sie enthalten pro Mol mehrere Monosaccharid-Einheiten, jedoch keinen PhosphorGehalt und werden den Sphingolipiden zugeordnet. Verglichen mit Proteinen sind sie eher niedermolekulare Biomoleküle. Die Ganglioside, an die die im Rahmen der vorliegenden Erfindung diskutierten Antikörper binden, sind das allgemein als G_{M1} bezeichnete Monosialo-Gangliosid bzw. die zugehörige "Asialo"-verbindung AG_{M1}. G_{M1} weist eine Polysaccharidkette aus 4 Zucker-Monomereinheiten auf, die zwei D-Galactoseeinheiten, eine N-Acetylgalactosamin- und eine D-Glucoseeinheit umfassen, wobei letztere an einen Ceramidteil gebunden ist. An die innerhalb der Polysaccharidkette angeordnete D-Galactoseeinheit ist bei dem Gangliosid G_{M1} ein N-Acetylneuraminsäurerest (NANA; Sialsäure- oder o-Sialinsäurerest; "Monosialo"-Rest) gebunden, der bei dem sialinsäurefreien Asialo-G_{M1} (AG_{M1}) fehlt.

Die genannten Ganglioside und verwandte Verbindungen werden mit zahlreichen wichtigen biologischen Funktionen des menschlichen Körpers in Zusammenhang gebracht, zu denen z.B. das exonale Wachstum und die neuronale Diffenzierung, Rezeptorenfunktionen und Beteiligungen an verschiedenen Immunreaktionen des Körpers sowie an der Signaltransduktion und 2e11-Zell-Erlcennung gehören. Es wird in diesem Zusammenhang beispielsweise verwiesen auf (14 bis 26).

Es ist seit langem bekannt, dass im menschlichen Körper Antikörper bzw. Autoantikörper auftreten können, die an die genannten und verwandte Ganglioside binden. Deren physiologische Rolle sowie ihre eventuelle Bedeutung für die klinische Diagnostik ist Gegenstand zahlreicher wissenschaftlicher Untersuchungen.

Der weitaus überwiegende Teil aller veröffentlichten Arbeiten befaßt sich mit der Rolle und der diagnostischen Signifikanz von Anti-Gangliosid-Antikörpern bei Neuropathien, z.B. bei immunvermittelten motorischen Neuropathien wie dem Guillain-Barre-Syndrom (Radikuloneuritis, Polyradikulitis) und dem verwandten (Miller-)Fisher-Syndrom (vgl. z.B. 27 bis 43; 63). Auch im Zusammenhang mit der Alzheimer-Erkrankung wurde bereits von einem vermehrten Auftreten von Anti-G_{M1-}Autoantikörpern bei einigen Patienten berichtet (42). Ferner wurden sie bei einzelnen HIV-Patienten gefunden (55 bis 57). Von einzelnen Versuchen ihrer Bestimmung im Zusammenhang mit bestimmten Krebsarten wurden ebenfalls berichtet (58 bis 60), wobei jedoch nur wenig aussagekräftige Ergebnisse bzw. Ergebnisse mit einer niedrigen Sensitivität erhalten wurden, worauf nachfolgend noch genauer einzugehen sein wird.

Studiert man die einschlägigen Veröffentlichungen genauer, fällt bei aller Ähnlichkeit der Beobachtungen auf, dass die Befunde und Aussagen bezüglich der zu beobachtenden Mengen - die in der Regel eher gering sind - und Typen der verschiedenen (Auto)-Antikörper bei den verschiedenen Patienten im einzelnen relativ stark voneinander abweichen. Das führte bereits zu dem Schluss, dass die Bestimmung derartiger Antikörper für die klinische Diagnostik nur von einem begrenzten bis zweifelhaften Wert sei (39, 62).

Bei der Anmelderin entstand aufgrund der z.T. erheblich divergierenden Literaturdaten der Eindruck, dass ein Grund dafür im Methodologischen liegen könnte und aufgrund systematischer Fehler der angewandten Meßmethoden bisher noch keine wirklich zuverlässigen, aussagekräftigen Ergebnisse vorliegen. Die meisten der Bestimmungen, deren Ergebnisse veröffentlicht wurden, wurden mit Immunoassays vom ELISA-Typ durchgeführt, die so gestaltet waren, dass man mit einer Festphase arbeitete, an die - teilweise von den Autoren selbst aus biologischem Material gewonnene - Ganglioside gebunden waren. Diese Festphase wurde mit der flüssigen biologischen Probe umgesetzt, in der die zu bestimmenden Antikörper vermutet wurden. Nach der jeweils gewählten Inkubationszeit, einer Fest-Flüssig-Trennung und einem Waschen der Festphase wurden dann an diese gebundene humane Antikörper unspezifisch mit enzymmarkierten tierischen anti-human-Ig-Antikörpern markiert und bestimmt.

Ein solches Assayschema ist bei seiner Anwendung auf die Anti-Gangliosid-Antikörper-Bestimmung außerordentlich anfällig für Störungen und Messfehler und kann nur bei sorgfältiger Standardiseriung und Normierung zuverlässige, reproduzierbare Ergebnisse liefern. Eine der Ursachen ist, dass die Qualität der Festphase, die durch Immobilisierung der relativ niedermolekulargewichtigen Ganglioside erhalten wird, anfällig für starke Schwankungen ist. Das ist teilweise dadurch begründet, dass vor der Umsetzung mit der flüssigen Probe restliche freie Bindungskapazitäten der Festphase abgesättigt werden müssen. Hierzu wird in der Regel Rinderserumalbumin, d.h. ein Protein verwendet. Dieser Schritt führt jedoch dazu, dass die unspezifische Bindung anderer Proteine, z.B. solcher vom IgG-Typ, aus der Probe sehr hoch wird, was zu einem starken Hintergrundsignal führt, vor dem die zu bestimmenden Antikörper bestimmt werden müssen. Ist jedoch die Empfindlichkeit eines Assays nicht sehr hoch - was bei Assays von ELISA-Typ in der Regel der Fall ist - können sich Hintergrundsignal und Messsignal so stark überlagern, dass unrichtige (falsch negative bzw. falsch positive) bzw. nicht zuverlässig reproduzierbare Messergebnisse erhalten werden. Zu den verschiedenen angewandten Assaymethoden und den systematischen und praktischen Problemen bei der Anwendung derartiger Methoden auf die Bestimmung von anti-Gangliosid-Antikörpern kann z.B. verwiesen werden auf (64 bis 68), wobei sich insbesondere (66) intensiver mit der Problematik der praktischen Anti-Gangliosid-Antikörperbestimmung befaßt.

Angesichts dieser Ausgangslage hatte sich die Anmelderin entschieden, das Problem der reproduzierbaren Bestimmung von anti-G_{M1}-bzw. anti-AG_{M1}-Antikörpern und der diagnostischen Signifikanz einer solchen Bestimmung z.B. bei Alzheimer-Patienten aufzugreifen und dabei die bei ihr als Produzentin von Assays für die klinische Diagnostik von Autoantikörpern vorhandenen besonderen Erfahrungen und Mittel zu nutzen. Für die interne Forschung entwickelte sie unter Einhaltung aller üblichen Qualitätsstandards Varianten einer verbesserten Modifikation der vorbekannten Antigangliosidassays. Die mit diesen verbesserten Assays durchgeführten Messungen von an G_{M1} bzw. AG_{M1} bindenden Antikörpern in Seren eines Vergleichskollektivs von Normalpersonen (Blutspendern) ohne relevante klinische Krankheitsbefunde sowie in Seren verschiedener Erkrankter lieferte, wie weiter unten ausgeführt wird, das überraschende Ergebnis, dass in allen der Anmelderin zur Verfügung stehenden Seren von krebskranken Patienten gegenüber Normalpersonen hoch signifikant erhöhte Titer für anti-G_{M1}- bzw. anti-AG_{M1}-Antikörper vom IgA sowie vom IgG-Typ, dagegen nicht vom IgM-Typ, gefunden wurden, und zwar mit einer Sensitivität, die nahezu 100% erreichte, und dass diese signifikant erhöhten Titer in allen Krebsseren gefunden wurden, ohne dass eine Selektivität bezüglich irgendeiner bestimmten Krebserkrankung erkennbar wurde. Dieser Befund war außerordentlich überraschend und steht im Gegensatz zu allen bisherigen Erfahrungen mit als Tumormarker untersuchten bzw. eingesetzten Biomolekülen.

Obwohl die nachfolgend genauer beschriebenen Ergebnisse mit einem bestimmten verbesserten Ligandenbindungsassay ("Immunoassay") aus dem Labor der Anmelderin gewonnen wurden, ist die Nutzung der dabei gewonnenen Erkenntnisse nicht nur mit einem Assay des beschriebenen speziellen Formats möglich. Es wird vielmehr davon ausgegegangen, dass der nachfolgend beschriebene konkrete Assay für die in Frage stehende Antikörperbestimmung noch deutlich suboptimal ist, und dass kommerzielle Assays für die klinische Bestimmung von Anti-Gangliosid-Antikörpern, insbesondere von anti-G_{M1}- und anti-AG_{M1}-(Auto)antikörpern sich von dem beschriebenen Assay nach einer Optimierung in mehrfacher Hinsicht noch deutlich unterscheiden werden.

Die Verfahren zur Bestimmung der genannten Antikörper in einer biologischen Probe können beliebige bekannte Verfahren der Immundiagnostik sein, die zum Nachweis und zur Messung von Antikörpern (Autoantikörpern) angewandt werden. Vorzugsweise werden die Antikörper mit Hilfe eines Ligandenbindungsassays bestimmt, bei dem man als Antigen zur Bindung der gesuchten Antikörper das jeweilige Gangliosid in immobilisierter Form verwendet. Zur Markierung der aus einer biologischen Probe spezifisch gebundenen Antikörper kann man dann auf irgendeine geeignete, an sich bekannte Weise markierte Anti-Human-Antikörper, markierte Ganglioside oder deren Kohlenhydratstruktur simulierende Binder mit einer für das jeweilige Assayformat geeigneten Affinität verwenden.

Auch kompetitive Assayformate können besondere Vorteile bieten. Vorzugsweise wird dabei nicht mit einer Enyzmmarkierung gearbeite, sondern es wird eine andere Markierung gewählt, z.B. eine Markierung für eine Chemilumineszenz-Nachweisreaktion, z.B. ein Akridiniumester. Selbstverständlich ist vorzugsweise ein solcher Assay zur Antikörperbestimmung zu verwenden, der die benötigte hohe Sensitivität im Bereich der vorkommenden Antikörper-Konzentrationen gewährleistet und eine Separierung der Messignale vom Assay-Hintergrund ermöglicht.

Das Bestimmungsverfahren kann an die Chip-Technologie angepaßt sein oder als Schnelltest (Point-of-Care-Test) ausgestaltet werden.

Zur Vermeidung von ungerechtfertigt engen und einschränkenden Auslegungen der in der vorliegenden Anmeldung und den zugehörigen Ansprüchen verwendeten Begriffe sollen nachfolgend einige der wichtigsten Begriffe für die Zwecke der vorliegenden Anmeldung besonders definiert werden:

| | |
|---|---|
| "Antikörper": | Dieser Begriff umfaßt, ohne zwischen verschiedenen Entstehungs- und Bildungsarten zu unterscheiden, Antikörper sowohl gegen externe Antigene als auch gegen körpereigene Strukturen, d.h. Autoantikörper, wobei letztere ggf. auch durch Antigen-Kreuzreaktionen aus Antikörpern gegen externe Antigene zu Autoantikörpern geworden sein können und ihre Bindungsfähigkeit gegen externe Antigene bewahrt haben können. |
| | |
| | Wenn z.B. davon gesprochen wird, dass ein Antikörper "an Gangliosidstrukturen und an Gangliosidstrukturen simulierende Antigenstrukturen" bindet oder gegenüber "Gangliosiden bzw. bestimmten Gangliosiden reaktiv" |
| | ist, wobei reaktiv "im Sinne einer spezifischen Bindung reaktiv" bedeutet, soll er durch diese Definition hinreichend definiert sein, ohne dass z.B. seine spezifische Bindung auch an zusätzliche andere antigene Strukturen, oder seine praktische Bestimmung unter Verwendung von Reagenzien (zur Immobilisierung bzw. Markierung bzw. als Kompetitoren) mit molekularen Strukturen, die AG_{M1}, insbesondere dessen Kohlenhydratstuktur, nur simulieren, für die Definition als erfindungsgemäßer Antikörper eine Rolle spielen sollen. |
| | |
| "Assay" | Dieser Begriff umfaßt beliebige für eine Bestimmung der fraglichen (Auto-)Antikörper geeignete hoch sensitive Ligandenbindungsassays, ohne dass eine Beschränkung auf ein bestimmtes Assayformat (Sandwichassay, kompetitiver Assay, Agglutinationsassay) oder eine bestimmte Art der Markierung gewünscht wird. Es versteht sich, dass bestimmte Assayformate und/oder Markierungen anderen überlegen und daher bevorzugt sind (z.B. eine Chemilumineszenz- gegenüber einer Enzymmar- |
| | kierung). Die Verwendung eines gegenüber dem nachfolgend konkret beschriebenen Assay verschlechterten oder verbesserten Assays soll jedoch nicht aus dem Bereich der Ansprüche herausführen, wenn er den in der vorliegenden Anmeldung definierten diagnostischen Zwecken dient. |
| | |
| "Sensitivität" | Eine hohe Sensitivität bedeutet im Rahmen der vorliegenden Erfindung, dass die Antikörper bei mindestens 50%, besser 70%, vorzugsweise mindestens 85% und nach stärker bevorzugt mindestens 95% aller an Krebs Erkrankten gefunden werden. |
| | |
| "universell" | Dieser Begriff bedeutet, wie der Begriff "unspezifisch", in Verbindung mit Begriffen wie "Biomarker" oder "Krebs- oder Tumormarker", dass keine Spezifität für eine bestimmte Krebserkrankung besteht und der Marker bei im wesentlichen allen Krebserkrankungen, wenigstens bei allen üblichen Krebserkrankungen der inneren Organe und metastasierenden Krebserkrankungen, und ganz besonders bei den in der nachfolgend in Tabelle 5 unter den Ziffern 1 bis 13 genannten Krebserkrankungen in diagnostisch signifikanten Mengen gefunden wird. |

Weitere Begriffsbedeutungen ergeben sich für den Fachmann aus der einleitenden und nachfolgenden Beschreibung der Erfindung und ihrer Ausführungsbeispiele.

In der nachfolgenden Beschreibung wird, zusätzlich zu dem Inhalt einiger Tabellen, auch auf Figuren Bezug genommen, die zeigen:
- Fig. 1: eine graphische Darstellung der Ergebnisse der Messung von Antikörpern der IgG-Klasse, die an Monosialo-G_{M1} binden, in Seren von 137 Kontrollpersonen, gegenübergestellt den Ergebnissen der Vermessung von Seren von 147 Tumorpatienten;
- Fig. 2: die Ergebnisse einer Vermessung der gleichen Seren wie in Fig. 1 auf Antikörper der IgA-Klasse, die an Monosialo-G_{M1} binden;
- Fig. 3: die Ergebnisse der Bestimmung von Antikörpern der IgG-Klasse, die an Asialo-G_{M1} binden, in Seren von 30 Normalpersonen (Kontrollen), gegenübergestellt den Ergebnissen der Vermessung von 30 Tumorseren (alle Seren sind Teilkollektive der in den Figuren 1 und 2 vermessenen Seren);
- Fig. 4: die Ergebnisse der Bestimmung von Antikörpern der IgA-Klasse, die an Asialo-G_{M1} binden, in den gleichen Seren wie in Fig. 3;
- Fig. 5: ein Diagramm, in dem eine Aufschlüsselung der Antikörperbestimmung in den Tumorseren gemäß Fig. 1 bezüglich der klinisch diagnostizierten Tumorarten/Erkrankungen erfolgt, die durch Zahlen von 1 bis 14 gekennzeichnet sind, wobei die Bedeutung der Zahlen 1 bis 14 in der nachfolgenden Beschreibung erläutert wird;
- Fig. 6: eine der Aufschlüsselung von Fig. 5 entsprechende Aufschlüsselung der Messung von Antikörpern der IgA-IClasse gemäß Fig. 2;
- Fig. 7: eine der Aufschlüsselung von Fig. 5 entsprechende Aufschlüsselung der Messung von Antikörpern der IgA-Klasse gemäß Fig. 3;
- Fig. 8: eine der Aufschlüsselung von Fig. 5 entsprechende Aufschlüsselung der Messung von Antikörpern der IgA-Klasse gemäß Fig. 4;
- Fig. 9: ein Diagramm, in dem eine Korrelation der Ergebnisse der Bestimmungen, im gleichen Seren-Teilkollektiv, von Antikörpern vom IgG-Typ, die an Monosialo-G_{M1} binden, und der Bestimmung von Antikörpern vom IgG-Typ, die an Asialo-G_{M1} binden, hergestellt wird (vgl. Fig. 1 und Fig. 3); und
- Fig. 10: eine Korrelation entsprechend der Fig. 9 der Bestimmung von Antikörpern der IgA-Klasse gemäß Fig. 2 und Fig. 4.

### Antikörper-Bestimmungen:

### 1. Herstellung der Assaykomponenten:

### A. Herstellung von Teströhrchen (Coated Tubes; CT)

Es wurden drei Arten von Teströhrchen hergestellt: (a) Teströhrchen, an die die Ganglioside G_{M1} bzw. AG_{M1} gebunden waren, sowie (b) Teströhrchen mit einer BSA-Beschichtung zur Bestimmung des probenindividuellen Hintergrundsignals.
a) Zur Herstellung der gangliosidbeschichteten Teströhrchen (GA-CTs) wurden die Ganglioside (G_{M1} und AG_{M1}, jeweils bezogen von der Fa. Sigma, Deutschland) in Methanol gelöst und anschließend in PBS (phosphatgepufferte Salzlösung), pH 7,2, 25% Methanol, auf eine Konzentration von 5µg/ml verdünnt. Von dieser Lösung wurden jeweils 300 µl in Polystyrolröhrchen (Polystyrolröhrchen "Star" der Fa. Greiner, Deutschland) gegeben und bei Raumtemperatur für 16h inkubiert. Anschließend wurde der Inhalt der Röhrchen durch Absaugen entfernt, und die Röhrchen wurden zur Absättigung freier Bindungsstellen mit 4,5 ml 0,5% BSA (bovines Serumalbumin, proteasefrei, Fa. Sigma, Deutschland) in Wasser befüllt und 2h bei Raumtemperatur inkubiert. Dann wurde der Röhrcheninhalt dekantiert, und die Röhrchen wurden mit 0,2% Tween, 10 mM Tris/HCl, 10 mM NaCl, pH 7,5 befüllt und wieder dekantiert. Anschließend wurden die Röhrchen zur Antikörperbestimmung eingesetzt.
(b) Da Serumbestandteile an das zur Absättigung freier Bindungsstellen der Teströhrchenwand verwendete BSA binden, und der Grad einer solchen Bindung bei unterschiedlichen Seren sehr unterschiedlich sein kann, ist es erforderlich, für jedes Serum getrennt ein probenindividuelles Hintergrundsignal zu bestimmen.

Zu diesem Zweck wurden die gleichen Teströhrchen mit 4,5 ml 0,5% BSA in Wasser befüllt und 2h bei Raumtemperatur inkubiert. Dann wurde der Röhrcheninhalt dekantiert, und die Röhrchen wurden mit 0,2% Tween, 10 mM Tris/HCl, 10 mM NaCl, pH 7,5 befüllt und wieder dekantiert. Anschließend wurden die Röhrchen (HR-CTs) zur Bestimmung des probenindividuellen Hintergrundsignals eingesetzt.

### B. Herstellung von Akridiniumester-markierten anti human-IgG bzw. anti human-IgA Antikörpern (Tracer)

Ziegen anti human-IgG Antikörper (affinitätsgereinigt; grade II, Fa. Scantibodies, USA) bzw. Ziegen anti human-IgA Antikörper (affinitätsgereinigt; Fa. Sigma, Deutschland), jeweils 2 mg/ml in PBS, pH 7,4, 100 µl wurden mit je 10 µl Akridinium-NHS-ester (Fa. Hoechst, Deutschland; 1 mg/ml in Acetonitril; vgl. DE 36 28 573 A1) gemischt und für 20 min bei Raumtemperatur inkubiert. Nach Zusatz von 300 µl 20 mM Glycin, 50 mM NaCl, wurden die markierten Antikörper mittels Adsorptionschromatographie über Hydroxylapatit-HPLC gereinigt. Als Trennsäule wurde eine HPHT-Säule (120 mm x 8 mm) verwendet, äquilibriert in Lösemittel A (1 mM NaPO₄, pH 7,0, 10% Methanol, 0,1% Lubrol; "LM A"; Lubrol 17A17 wurde von der Fa. Serva, Deutschland bezogen). Die Durchflussgeschwindigkeit betrug 0,8 ml/min. Gebundene Antikörper wurden mittels eines linearen 40 min-Gradienten aus LM A/LM B (500 mM NaPO₄, pH 7,0, 10% Methanol, 0,1% Lubrol; "LM B") bei einer Flussgeschwindigkeit von 0,8 ml/min eluiert. Der Säulenausfluss wurde kontinuierlich auf UV-Absorption bei 280 nm (Protein) und 368 nm (Akridiniumester) vermessen. Nicht proteingebundener Akridiniumester wurden ungebunden aus der Säule eluiert und damit vollständig von den markierten Antikörpern abgetrennt. Die Antikörper wurden bei ca. 25 min eluiert. Nach der Bestimmung der Proteinkonzentration (BCA Methode) der HPLC-gereinigten markierten Antikörper erfolgte eine Verdünnung der Tracer auf eine Endkonzentration von 0,1 µg/ml in PBS, pH 7,2, 1 mg/ml Ziegen-IgG (Fa. Sigma, Deutschland) und 1% BSA.

### 2. Durchführung der Bestimmung von anti-Gangliosid Antikörpern

Zu untersuchende Proben (Humanseren) wurden 1:20 mit PBS, pH 7,2, 1 mg/ml Ziegen IgG, 1% BSA verdünnt. Hiervon wurden jeweils 10 µl in GA-CTs bzw. HR-CTs pipettiert. Anschließend erfolgte eine 16h Inkubation unter Schütteln (IKA-Schüttler KS250 basic, 400 U/min) bei 4°C.

Ungebundene Antikörper wurden durch 5maliges Befüllen/Dekantieren der Röhrchen mit 1 ml 0,2% Tween, 10mM Tris/HCl, 10 mM NaCl, pH 7,5 entfernt. Auf den Röhrchenoberflächen verbliebene Antikörper wurden durch Bindung markierter Ziegen anti human-IgG bzw. markierter Ziegen anti human-IgA nachgewiesen, indem man die Röhrchen mit jeweils 200 µl des jeweiligen Tracers (vgl. oben, 1.B.) und anschließend für 3h bei 4°C unter Schütteln inkubiert. Ungebundener Tracer wurde durch 5maliges Waschen (wie oben) entfernt.

Die Menge des auf der Röhrchenoberfläche verbliebenen markierten Antikörpers wurde mittels Lumineszenzmessung in einem Berthold LB.952T/16 Luminometer gemessen.

Das für GA-CTs erhaltene Lumineszenzsignal einer jeden Probe wurde dabei um das mit den HR-CTs gemessene jeweilige Hintergrundsignal für die gleiche Probe korrigiert. Das resultierende Signal (Differenzsignal) ist das Signal für an die Ganglioside G_{M1} bzw. AG_{M1} bindenden Antikörper aus der jeweiligen Probe. Als relative Kalibratoren für die Quantifizierung wurden Verdünnungsreihen von Proben mit einem hohen Gehalt an anti-Gangliosid-Antikörpern verwendet.

### 3. Vermessung von Seren von gesunden Normalpersonen (Kontrollen) und Krebspatienten

Unter Verwendung der wie beschrieben hergestellten Teströhrchen und unter Anwendung des oben beschriebenen Verfahrens wurden die folgenden Reihenmessungen vorgenommen:

Kontrollseren: Als Kontrollseren für die Antikörper-Bestimmungen unter Verwendung von GA-CTs, die mit G_{M1} beschichtet waren, dienten 137 Kontrollseren (Blutspenderseren und zur Vermeidung von Lebensalter-bedingten Einflüssen auf die Antikörperkonzentrationen Seren von Normalpersonen aus Pflegeheimen verschiedenen Alters und von Mitarbeitern der Anmelderin). Für die Antikörper-Bestimmungen unter Verwendung von GA-CTs, die mit AG_{M1} beschichtet waren, wurde ein Teilkollektiv dieser Seren vermessen, das nur 30 Seren umfaßte.

Testseren: Als Testseren für die Antikörper-Bestimmungen unter Verwendung von GA-CTs, die mit G_{M1} beschichtet waren, dienten 147 Seren von Patienten mit klinisch diagnostizierten Tumoren verschiedener Organe/Gewebe sowie, zusätzlich, 20 Seren von Patienten mit chronisch entzündlichen Darmerkrankungen. Zu jedem Testserum existierte eine genaue klinische Dokumentation, die eine Aufschlüsselung der in die Messung eingesetzten Seren von Krebspatienten nach der bei ihnen festgestellten Tumorart ermöglichte. Für die Antikörper-Bestimmungen unter Verwendung von GA-CTs, die mit AG_{M1} beschichtet waren, wurde ein Teilkollektiv dieser Seren vermessen, das nur 30 Seren von Krebspatienten umfaßte.

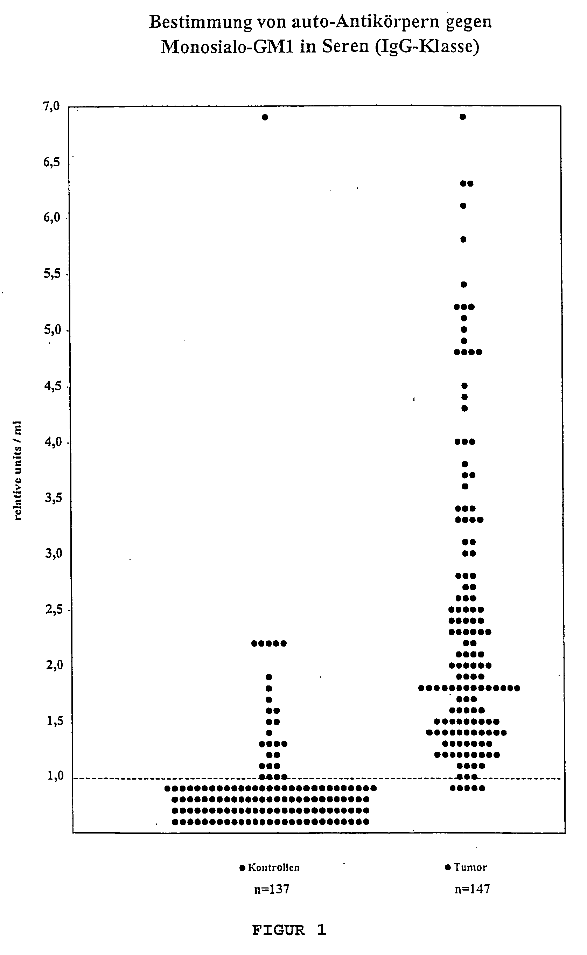

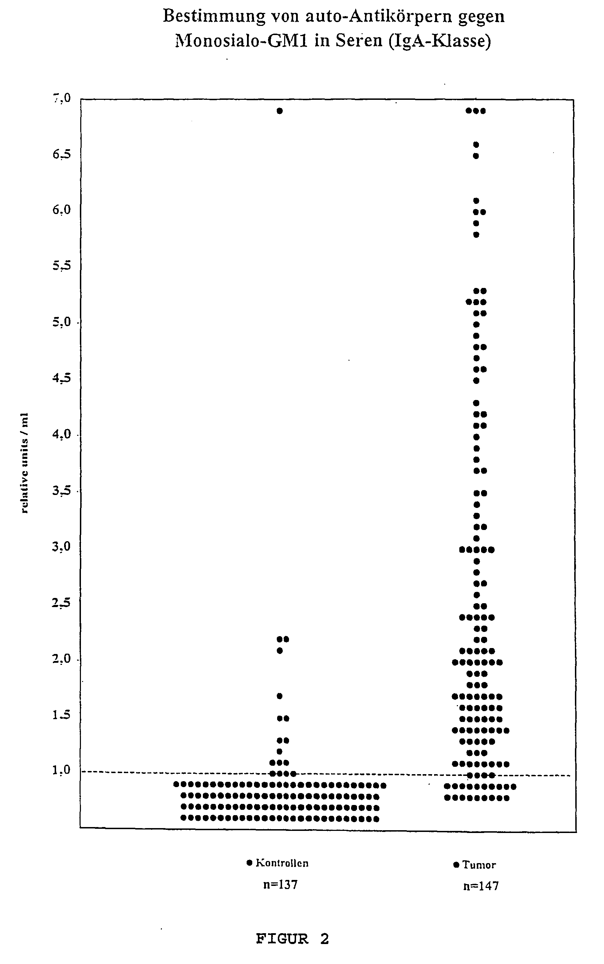

Die Ergebnisse der Bestimmungen von Antikörpern der Klassen IgG und IgA unter Verwendung von GA-CTs, die mit G_{M1} beschichtet waren, sind in den Figuren 1 und 2 gezeigt.

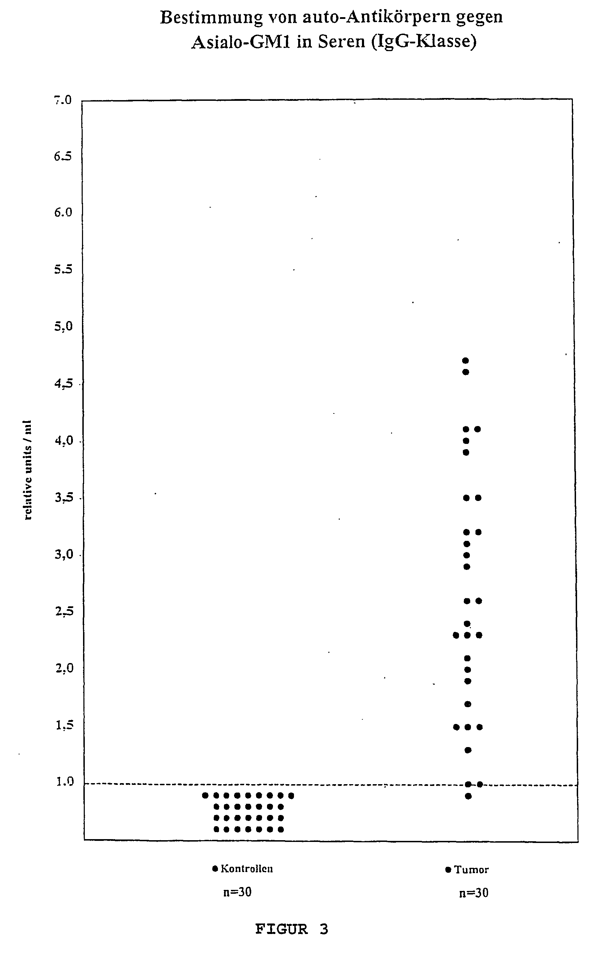

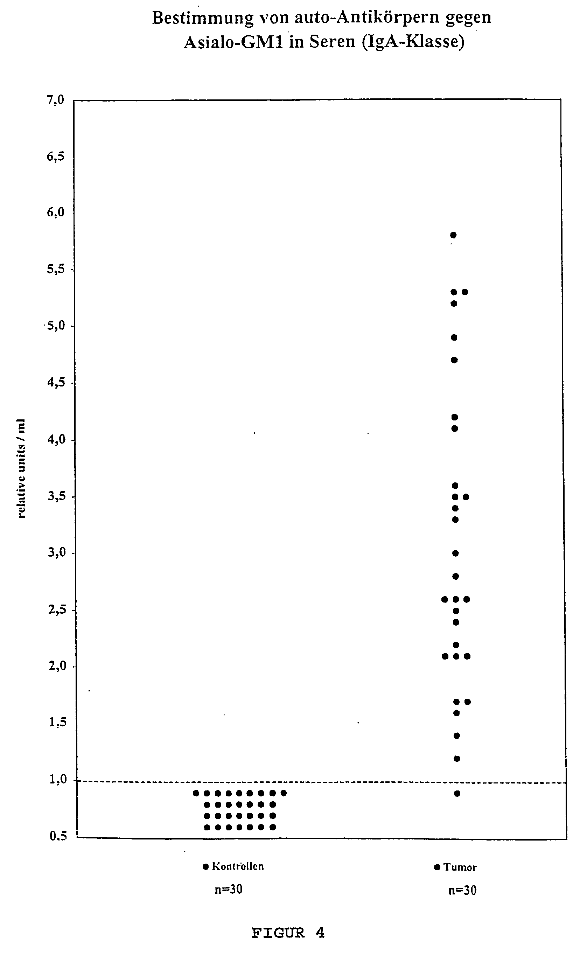

Die Ergebnisse der Bestimmungen von Antikörpern der Klassen IgG und IgA unter Verwendung von GA-CTs, die mit AG_{M1} beschichtet waren, sind in den Figuren 3 und 4 gezeigt.

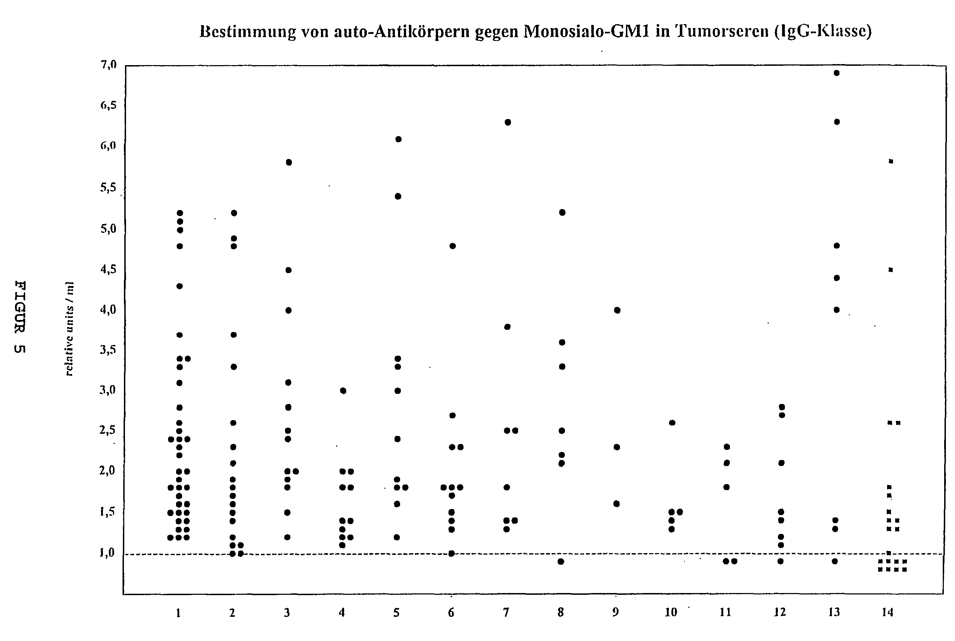

Figur 5 zeigt eine Aufschlüsselung der Messergebnisse gemäß Fig. 1 (IgG) nach Krankheitsbildern. Dabei stehen die in waagerechter Richtung aufgetragenen Kennziffern für die der folgenden Aufstellung entnehmbaren klinischen Diagnosen:

| Kennziffer | Diagnose | Zahl der Seren |
|---|---|---|
| 1 | Colon-Karzinom | n = 37 |
| 2 | Mamma-Karzinom | n = 19 |
| 3 | Ovarial-Karzinom | n = 13 |
| 4 | Magen-Karzinom | n = 11 |
| 5 | Pankreas-Karzinom | n = 11 |
| 6 | Ösophagus-Karzinom | n = 12 |
| 7 | Gallen-Karzinom | n = 8 |
| 8 | Leber-Karzinom | n = 7 |
| 9 | C-Zell-Karzinom | n = 3 |
| 10 | Schilddrüsen-Karz. | n = 5 |
| 11 | Prostata-Karzinom | n = 5 |
| 12 | Lungen-Karzinom | n = 8 |
| 13 | Sonstige Karzinome | n = 8 |
| 14 | M.Crohn/C.Ulcerosa | n = 20 |

Die "Sonstigen Karzinome" teilten sich dabei wie folgt auf:

Appendix-Karzinom (n=1), Blasen-Karzinom (n=1), Cardia-Karzinom (n=1), Distales Verophagus-Karzinom (n=2), Mundboden (n=1), Nieren-Karzinom (n=1), Oberbauchtumor (n=1).

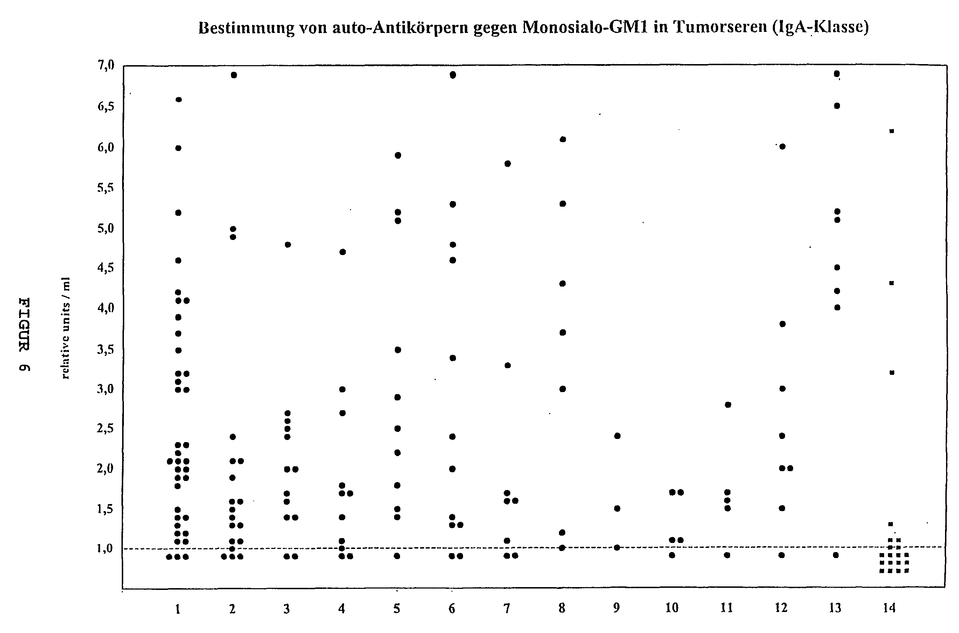

Figur 6 ist eine entsprechende Aufschlüsselung der Ergebnisse von Fig.2 (IgA).

Die nachfolgende Tabelle 1 faßt die Ergebnisse der Figuren 5 und 6 zahlenmäßig zusammen.

**Tabelle 1**

| Betroffenes Organ | anti G_{M1} IgG | anti GM1 IgA | n |
|---|---|---|---|
| Colon/Rektum | 38 = 100% | 35 = 92% | 38 |
| Mamma | 19 = 100% | 16 = 84% | 19 |
| Ovarien | 13 = 100% | 11 = 85% | 13 |
| Magen | 11 = 100% | 9 = 82% | 11 |
| Pankreas | 11 = 100% | 10 = 91% | 11 |
| Ösophagus | 12 = 100% | 10 = 83% | 12 |
| Galle | 8 = 100% | 6 = 75% | 8 |
| Leber | 6 = 86% | 7 = 100% | 7 |
| C-Zell | 3 = 100% | 3 = 100% | 3 |
| Lunge/ Bronchien | 7 = 88% | 7 = 88% | 8 |
| Schilddrüse | 5 = 100% | 4 = 80% | 5 |
| Prostata | 3 = 60% | 4 = 80% | 5 |
| sonstige | 7 = 88% | 7 = 88% | 8 |

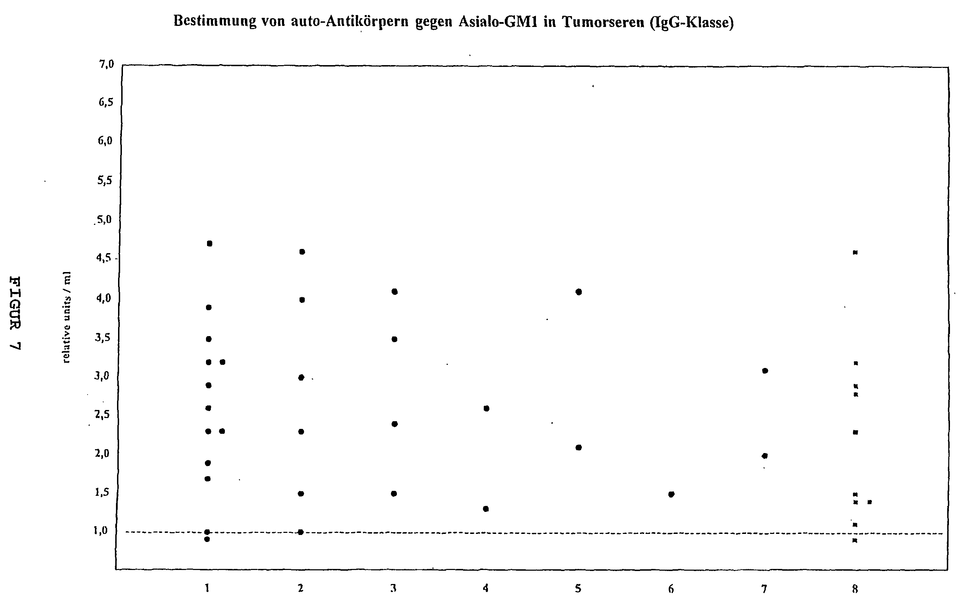

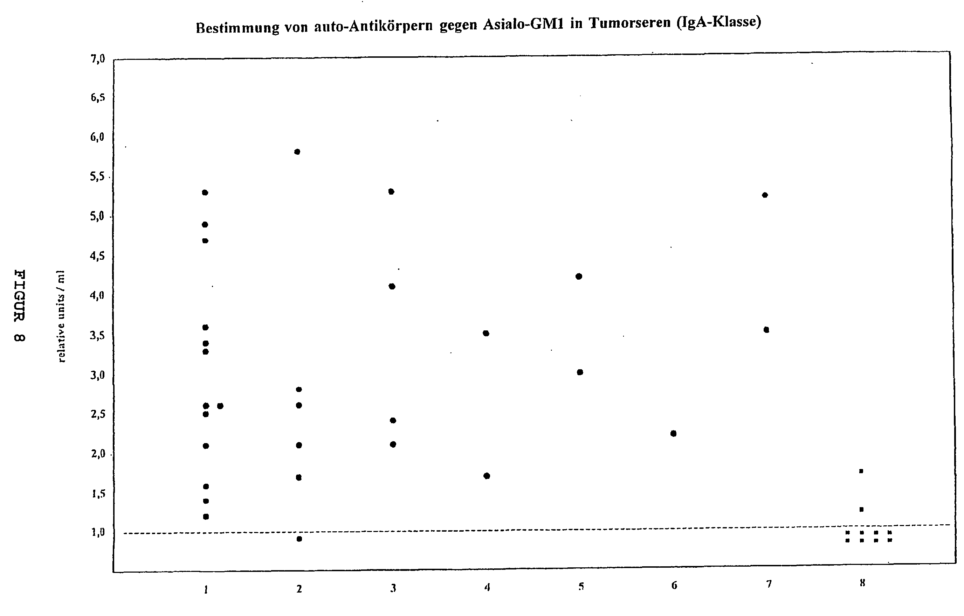

Die Figuren 7 und 8 sind den Figuren 5 und 6 entsprechende Aufschlüsselungen der mit AG_{M1}-beschichteten GA-CTs erhaltenen Ergebnisse für die vermessenen Teilkollektive gemäß den Figuren 3 und 4. Dabei stehen in den Figuren 7 und 8 die Kennziffern für die folgenden Diagnosen:

| Kennziffer | Diagnose | Zahl der Seren |
|---|---|---|
| 1 | Colon-Karzinom | n = 13 |
| 2 | Mamma-Karzinom | n = 6 |
| 3 | Pankreas-Karzinom | n = 4 |
| 4 | Ösophagus-Karzinom | n = 2 |
| 5 | Gallen-Karzinom | n = 2 |
| 6 | Lungen-Karzinom | n = 1 |
| 7 | Sonstige: | n = 2 |
| | Oberbauchtumor | n = 1 |
| | Appendix-Karzinom | n = 1 |
| 8 | M.Crohn/C.Ulcerosa | n = 10 |

Die nachfolgende Tabelle 2 faßt die Ergebnisse der Figuren 7 und 8 zahlenmäßig zusammen.

**Tabelle 2**

| Betroffenes Organ | anti AG_{M1} IgG | anti AG_{M1} IgA | n |
|---|---|---|---|
| Colon/Rektum | 12 = 92% | 13 = 100% | 13 |
| Mamma | 6 = 100% | 5 = 83% | 6 |
| Pankreas | 4 = 100% | 4 = 100% | 4 |
| Ösophagus | 2 = 100% | 2 = 100% | 2 |
| Galle | 2 = 100% | 2 = 100% | 2 |
| Leber | 1 = 100% | 1 = 100% | 1 |
| Lunge/ Bronchien | 1 = 100% | 1 = 100% | 1 |
| sonstige | 2 = 100% | 2 = 100% | 2 |

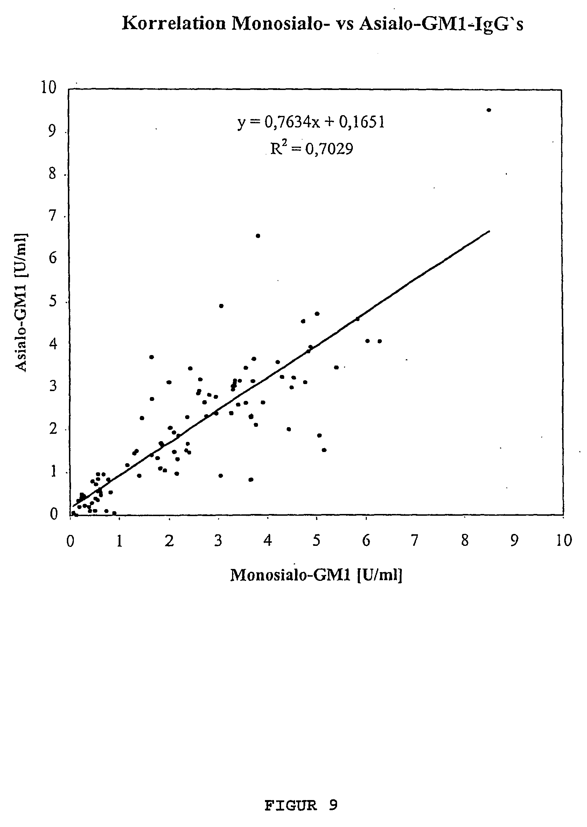

Führt man eine quantitative Korrelation der einerseits mit G_{M1}-beschichteten Teströhrchen und andererseits, für das gleiche Seren-Teilkollektiv, mit AG_{M1}-beschichteten Teströhrchen erhaltenen Messergebnisse für die IgG-Bestimmung durch, so ergibt sich, wie in Figur 9 gezeigt ist, eine weitgehende Übereinstimmung, die den Schluss erlaubt, dass bei beiden Bestimmungen wenigstens weitestgehend identische Antikörper gemessen wurden.

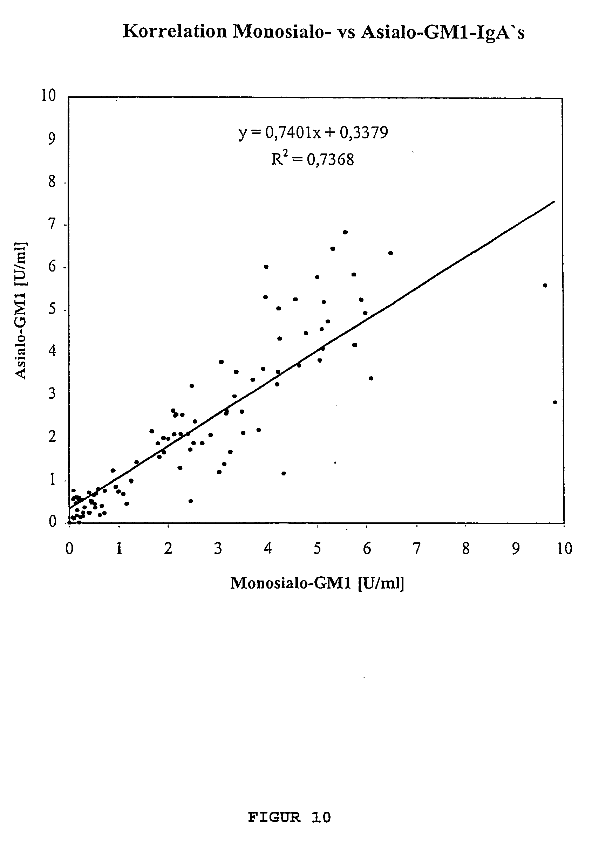

Ähnliches gilt für die IgA-Bestimmungen, wobei die Figur 9 entsprechende Korrelation in Figur 10 gezeigt ist.

### 4. Diskussion der Befunde der Bestimmung von anti-Gangliosid-Antikörpern in Kontrollseren und in Seren von Krebspatienten

Wie die in den Figuren 1 bis 10 und den obigen Tabellen 1 und 2 zusammengefassten Messergebnisse eindrücklich zeigen, ermöglicht die Bestimmung von Antikörpern, die an Ganglioside (AG_{M1} und/oder G_{M1}) binden, ein klare Unterscheidung der beiden Grupen von vermessenen Seren. Die Sensitivität der durchgeführten Bestimmungen lag für alle Tumorarten über 75%, in den meisten Fällen sogar bei 100%. Dabei scheint es, dass die Bestimmungen von IgA unter Verwendung von AG_{M1-}beschichteten Teströhrchen Meßergebnisse mit der höchsten Sensitivität liefern (wobei allerdings einschränkend zu berücksichtigen ist, dass die Zahl der Bestimmungen geringer war als im Falle der Verwendung von G_{M1}-beschichteten Röhrchen).

Es ist ferner darauf hinzuweisen, dass bei einem Versuch, entsprechend den Bestimmungen von Antikörpern vom IgG- und IgA-Typ auch solche vom IgM-Typ zu bestimmen, keine diagnostisch relevanten Ergebnisse erhalten wurden (Ergebnisse nicht gezeigt).

Die gemessenen hohen Sensitivitäten in Verbindung mit einer hohen Unspezifität bezüglich der verschiedenen Krebsarten machen die Bestimmung von anti-Gangliosid-Antikörpern, insbesondere von solchen der IgG- und/oder IgA-Klassen, zu einem vielversprechenden Bestimmungsverfahren für die Diagnose, insbesondere für die Früherkennung, von Neoplasmen.

Der wissenschaftlichen Literatur lassen sich Erkenntnisse, die ein solches Verfahren nahelegen könnten, nicht entnehmen. Es wurden nur einige wenige Arbeiten bekannt, in denen der Versuch unternommen wurde, im Zusammenhang mit Krebserkrankungen auch anti-Gangliosid-Antikörper zu bestimmen. Ausgehend von dem Befund, dass beim sogenannten "small cell lung cancer" (SCLC) im Lungengewebe von Erkrankten eine erhöhte/abnormale Expression von Gangliosiden gefunden wurde, wurde in zwei Arbeiten (58, 59) untersucht, ob diese abnormale Gangliosidexpression dazu führt, dass bei Krebspatienten anti-Gangliosid-Antikörper gebildet und nachgewiesen werden können. In (58) wurden die gesuchten anti-Fukosyl-GM1-Gangliosid-Antikörper nicht gefunden, während in (59) für den Fall von differenziertem Schilddrüsenkrebs (DTC) zwar in einer gewissen Anzahl von Patienten geringe Mengen an verschiedenen anti-Gangliosid-Antikörpern nachgewiesen werden konnten, jedoch z.B. im Falle der anti-GM1-Antikörper-Bestimmung die gefundenen Werte für die Krebspatienten unter denen der Kontrollen lagen (Fig.1). Nur im Hinblick auf eine Bindung an das fukosylierte Gangliosid FucGM1 wurden etwas stärker erhöhte Antikörpertiter gefunden. Im Lichte der in der vorliegenden Anmeldung beschriebenen Befunde, die denen gemäß (58, 59) klar widersprechen, ist anzunehmen, dass es den Autoren von (58, 59) angesichts der erheblichen praktischen Schwierigkeiten bei der Bestimmung von anti-Gangliosid-Antikörpern vor einem intensiven, serumabhängigen Hintergrundsignal mit dem von ihnen verwendeten ELISA-Assay nicht gelang, tatsächlich aussagekräftige Messergebnisse zu produzieren. (58, 59) stellen somit einen von dem erfindungsgemäßen Verfahren klar wegführenden Stand der Technik dar.

In (60) wird ferner von einer Bestimmung von verschiedenen Typen von Antikörpern in Seren von Magenkrebspatienten berichtet, und es wird eine eventuelle prognostische Rolle einer solchen Bestimmung diskutiert, wobei "prognostisch" im Sinne einer Prognose für den weiteren klinischen Verlauf einer bereits bestehenden und erkannten Krebserkrankung verwendet wird. Zur Antikörper-Bestimmung wird ein ELISA-Assay verwendet. Zu den Antikörpern, die gegenüber Kontrollen erhöht gefunden wurden, gehören auch sog. anti-G_{M1}-Antikörper, die allerdings nur mit einer Sensitivität von etwa 35% bei den Erkrankten gefunden wurden (gegenübergestellt einem Wert von 5% für Normalpersonen). Im Lichte von (60) und anderen Arbeiten der Autoren von (60) sind diese anti-Gangliosid-Antikörper nur eine von zahlreichen Arten von im Zusammenhang mit Krebs untersuchten Autoantikörpern, und aus den in (60) berichteten Daten läßt sich keinerlei besondere Eignung der Bestimmung derartiger Antikörper für die Diagnose, insbesondere die Früherkennung, und zur Bestimmung des Risikos eines Patienten, eine maligne Krebserkrankung zu entwickeln, ableiten. Eine solche Perspektive ergab sich erstmals aus den von der Anmelderin unter Einsatz ihrer großen Erfahrung auf dem Assaygebiet erhaltenen, vor dem Hintergrund des Standes der Technik außerordentlich überraschenden Messdaten.

Die Anmelderin ist aufgrund ihrer Messergebnisse sowie einer zusätzlichen intensiven Literaturstudie der Überzeugung, dass den bei Krebspatienten gefundenen erhöhten anti-Gangliosid-(Auto-)Antikörper-Titern eine ganz andere Bedeutung zukommt als z.B. Ausgangspunkt der Überlegungen der Autoren von (58, 59, 60) war: Es ist nämlich bekannt, dass die sogenannten "natürlichen Killerzellen" (NK-Zellen; cytotoxisch aktive Lymphozyten) an ihrer Oberfläche Asialo-G_{M1-}Strukturen aufweisen, an die anti-AG_{M1}-Antikörper spezifisch binden können, die dadurch die NK-Zellen desaktivieren und zerstören. Aktive NK-Zellen spielen eine außerordentlich wichtige Rolle im Rahmen der Immunabwehr des Menschen, indem sie z.B. alle entarteten körpereigenen Zellen, z.B. Krebszellen, abtöten, die aufgrund ihrer Entartung die Fähigkeit verloren haben, die NK-Zellen zu hemmen, die mit Ihnen in Kontakt kommen. Eine Beeinträchtigung (Hemmung, Abtötung) der normalen NK-Zellen, die deren selektiv-cytotoxische Eigenschaften aufhebt, führt daher dazu, dass im Verlaufe der natürlichen Lebensvorgänge entartete Zellen, insbesondere vom Tumorzell-Typ, nicht mehr ordnungsgemäß eliminiert werden. Aufgrund der verbesserten Überlebenschancen derartiger entarteter potentieller Tumorzellen können sich diese bei einer gestörten Funktion der NK-Zellen im Körper festsetzen, ungestört teilen und zu einem eigentlichen Tumor entwickeln. Es ist darauf hinzuweisen, dass es auf dem Gebiet von Tierexperimenten, bei den mit Versuchstieren gearbeitet wird, bei denen ein künstlicher Krebs erzeugt wird, bereits üblich ist, durch Gabe von anti-AG_{M1}-Antikörpern in Verbindugn mit einem krebsauslösenden Karzinogen oder einem Tumorkeim die Immunabwehr des Versuchstiers auszuschalten, so dass sich der - im Tiermodell gewünschte - experimentelle Krebs entwickeln kann (3 bis 13).

Ist bei einem menschlichen Individuum, z.B. aufgrund einer bakteriellen Exposition (z.B. Infektionen mit *Campylobacter jejuni* oder auch *Helicobacter pylori;* vgl. 44 bis 54), die Produktion von anti-Asialo-G_{M1}-Antikörpern einmal initiiert bzw. stark erhöht, entsteht eine Voraussetzung für eine potentielle Schädigung der NK-Zellen und damit der Immunabwehr mit der Folge eines erhöhten Risikos, dass sich entartete Tumorzellen zu einem Tumor entwickeln können. Es ist also davon auszugehen, dass die in allen Krebsseren, die im Rahmen der o.g. Bestimmungen vermessen wurden, signifikant erhöht gefundenen anti-AG_{M1}-Antikörpertiter weniger im Sinne der bekannten Tumormarker eine Folge des Vorliegens eines Neoplasmas (Tumors) sind, sondern eher eine Voraussetzung seines Entstehens. Im Laufe der Tumorentwicklung kann es dann, in Verbindung mit einer stimulierten NK-Zell-Aktivität, u.U. zu einem "Aufschaukeln" der Antikörpertiter kommen.

Da die mit Gangliosiden kreuzreagierenden Antikörper und die für deren Produktion erforderliche Prägung des Immunsystems bereits vor der Entwicklung eines Tumors vorhanden sein können, kann die Bestimmung von anti-AG_{M1}-Antikörpern somit erfindungsgemäß auch im Sinne der Ermittlung einer Disposition, d.h. als Bestimmung eines Krebs-Risiko-Markers, erfolgen. Es kann in diesem Zusammenhang vorteilhaft sein, eine solche Bestimmung nach einer in vivo Stimulierung der Antikörperbildung unter Verwendung unbedenklicher Stimulantien zu vorzunehmen. Angesichts der deutlich erhöht gefundenen IgA-Antikörper kann die Bestimmung ausdrücklich auch mit geeigneten Assays in Körpersekreten (z.B. Speichel, Schleim) erfolgen.

Es ist in diesem Zusammenhang von hohem Interesse, dass, wie insbesondere den Figuren 5 bis 8 zu entnehmen ist, erhöhte Titer an anti-AG_{M1}-Antikörpern auch bei einem Teil von Patienten gefunden werden, die an einer chronisch entzündlichen Darmerkrankung (Morbus Crohn, Colitis Ulcerosa) leiden. Es ist bekannt, dass z.B. bei Colitis Ulcerosa eine erhöhtes Risiko besteht, dass sich bei den Patienten im späteren Verlauf Darmkrebs entwickelt. Das Risiko beträgt etwa 40%. Der Befund, dass dieser prozentuale Wert in der Größenordnung der Werte liegt, die in Seren von Patienten mit chronisch entzündlichen Darmerkrankungen (Kennziffer 14 in den Figuren 5 und 6; Kennziffer 8 in den Figuren 7 und 8) für erhöhte anti-AG_{M1}-Titer gefunden werden, d.h. eine Parallität von statistische Krebsrisiko und einem Auftreten von anti-AG_{M1}-Antikörpern in den Seren einschlägig Erkrankter erkennbar wird, ist ein weiterer die o.g. Annahmen stützender Befund.

Die sich aufgrund er in dieser Anmeldung geschilderten Befunde ergebenden Konsequenzen für neuartige Verfahren zur Prävention, Hemmung und Therapie von Krebs sind Gegenstand einer gleichzeitig mit der vorliegenden Anmeldung eingereichten eigenen parallelen Patentanmeldung.

### Literaturliste:

1. http://www.medicine-worldwide.de/krankheiten/krebs/xxx.html, wobei /xxx zu ersetzen ist z.B. durch /allgemeines; /lungenkrebs; /brustkrebs; /prostatakrebs; /darmkrebs; /leukaemie; /corpuskarzinom;
2. Lothar Thomas (Herausgeber), Labor und Diagnose, 5. erw. Auflage, Kapitel 34: Tumormarker, S. 956-1019;
3. Hugh F. Pross et al., Role of Natural Killer Cells in Cancer, Nat Immun 1993; 12:279-292;
4. Lewis L. Lanier et al., Arousal and inhibition of human NK Cells, Immunological Reviews 1997, Vol. 155:145-154;
5. Yoichi Fuji et al., IgG Antibodies to AsialoGM1 Are More Sensitive than IgM Antibodies to Kill *in vivo* Natural Killer Cells and Prematured Cytotoxic T Lymphocytes of Mouse Spleen, Microbiol.Immunol. Vol. 34(6), 533-542, 1990;
6. Carmine M. Volpe et al., AsGM1 + NK Cells Prevent Metastasis of Invading LD-MCA-38 Tumor Cells in the Nude Mouse, J Surg Res 84, 157-161 (1999);
7. Susan D. Wilson et al., Correlation of Suppressed Natural Killer Cell Activity with Altered Host Resistance Models in B6C3F1 Mice, Toxicology and Applied Pharmacology 177, 208-218 (2001);
8. H.Yoshino et al., Natural killer cell depletion by anti-asialo GM1 antiserum treatment enhances human hematopoietic stem cell engraftment in NOD/Shi-scid mice; Bone Marrow Transplantation (2000) 26, 1211-1216;
9. N.Saijo et al., Analysis of Metastatic Spread and Growth of Tumor Cells in Mice with Depressed Natural Killer Activity by Anti-asialo GM1 Antibody or Anticancer Agents, J Cancer Res Clin Oncol (1984) 107: 157-163;
10. Sonoku HABU et al., Role of Natural Kiler Cells against Tumor growth in Nude Mice - A Brief Review, Tokai J Exp Clin Med., Vol.8, No.5, 6: 465-468, 1983;
11. Lewis L. Lanier, NK Cell Receptors, Annu. Rev. Immunol. 1998, 16: 359-93;
12. Theresa L. Whiteside et al., The role of natural killer celss in immune surveillance of cancer; Current Opinion in Immunology 1995, 7:704-710;
13. Tuomo Timonen et al., Natural killer cell-target cell interactions, Current Opinion in Cell Biology 1997, 9:667-673;
14. Jose Abad Rodriguez et al., Plasma Membrane Ganglioside Sialidase Regulates Axonal Growth and Regeneration in Hippocampal Neurons in Culture, J Neurosci 21(21):8387-8395, (2001);
15. Lindsey A. Miles et al., Gangliosides Interact Directly with Plasminogen and Urokinase and May Mediate Binding of These Fibrinolytic Components to Cells, Biochemistry 1989, 28, 9337-9343;
16. Haruyuki Qshima et al., Gangliosides can activate human alternative complement pathway, International Immunology, Vol.5, No.10, 1349-1351 (1993);
17. I.M.Dozmorov et al., Nanomolar Concentrations of Gangliosides Stimulate Primary Humoral Response, Biochemistry and Molecular Biology International, Vol. 42, No.1:57-63 (1997);
18. John L. Ryan et al., Possible role for glycosphingolipids in the control of immune responses, Eur.J.Immunol. 1979, 9:171-175;
19. Robert W. Ledeen et al., The Role of GM1 and Other Gangliosides in Neuronal Differentiation, Annals New York Academy of Sciences, 1998, 161-175;
20. Sen-itiroh Hakomori et al., Functional Role of Glycosphingolipids in Cell Recognition and Signaling, J.Biochem. 118, 1091-1103 (1995);
21. Allan J.Yates et al., Ganglioside modulation of the PDGF receptor, Journal of Neuro-Oncology, 24, 65-73, 1995;
22. Anup K. Singh et al., Gangliosides as Receptors for Biological Toxins: Development of Sensitive Fluoroimmunoassays Using Ganglioside-Bearing Liposomes, Analytical Chemistry, Vol.72, No.24:6019-6024 (2000);
23. Howard C. Krivan et al., Many pulmonary pathogenic bacteria bind specifically to the carbohydrate sequence GalNAcβ1-4Gal found in some glycolipids, Proc.Natl.Acad.Sci. USA, Vol.85:6157-6161 (1988);
24. Malin Bäckström et al., Characterization of an internal permissive site in the cholera toxin B-subunit and insertion of epitopes from human immunodeficiency virus-1, hepatitis B virus and enterotoxigenic *Escherichia coli,* Gene, 165 (1993) 163-171;
25. Wayne I. Lencer et al., Membrane traffic and the cellular uptake of cholera toxin, Biochimica et Biophysica Acta 1450 (1999) 177-190;
26. Adam J. Ratner et al., Cystic Fibrosis Pathogens Activate Ca²⁺-dependent Mitogen-activated Protein Kinase Signaling Pathways in Airway Epithelial Cells, J.Biol.Chem. Vol.276, No.22, 19267-19275 (2001);
27. Richard H. Quarles et al., Autoantibodies Associated with Peripheral Neuropathy, Muscle Nerve, July 1999, 800-822;
28. Isoardo G et al., Anti-GM1 and anti-sulfatide antibodies in polyneuropathies, Acta Neurol Scand 2001:103: 180-187;
29. Rayomand Press et al., Temporal profile of anti-ganglioside antibodies and their relation to clinical parameters and treatment in Guillain-Barré syndrome, J Neurol Sci 190 (2001) 41-47;
30. Eduardo Nobile-Orazio, Multifocal motor neuropathy, J. Neuroimmunol. 115 (2001) 4-18;
31. Angelo Quattrini MD et al., Human IgM anti-GM1 autoantibodies modulate intracellular calcium homeostasis in neuroblastoma cells; J. Neuroimmunol. 114 (2001) 213-219;
32. Edith Uetz-von Almen et al., Antiganglioside GM1 Antibodies and Their Complement Activating Capacity in Central and Peripheral Nervous System Disorders and in Controls, Eur Neurol 1998; 39:103-110;
33. Noboyuki Hirota et al., The physiologial effect of anti-GM1 antibodies on saltatory conduction and transmembrane currents in single motor axons, Brain (1997), 120, 2159-2169;
34. Amjad A. Ilyas et al., Anti-G_{M1} IgA antibodies in Guillain-Barre syndrome, J. Neuroimmunol., 36 (1992) 69-76;
35. Andrew J. Kornberg, Anti-GM1 ganglioside antibodies: their role in the diagnosis and pathogenesis of immunemediated motor neuropathies, J. Clin. Neurosci. (2000) 7(3), 191-194;
36. A.S.Bansal et al., IgM ganglioside GM1 antibodies in patients with autoimmune disease or neuropathy, and controls, J.Clin.Pathol. 1994; 47:300-302;
37. Carmen Garcia Guijo et al., Presence and isotype of anti-ganglioside antibodies in healthy persons, motor neuron disease, peripheral neuropathy, and other diseases of the nervous system; J. Neuroimmunol.56 (1995), 27-33;
38. Florina P. Thomas, Antibodies to GM1 and Gal(β1-3)Gal-Nac at the Nodes of Ranvier in Human and Experimental Autoimmune Neuropathy, Microscopy Research and Technique 34:536-543 (1996);
39. B.Gatterbauer et al., Antiglycosphingolipid Immune Responses in Neurology, Annals New York Academy of Sciences, 353-362;
40. H.J.Willison et al., Antiglycolipid antibodies, immunoglobulins and paraproteins in motor neuron disease: a population based case-control study; J.Neurol.Sci., 114 (1993) 209-215;
41. Chowdury D, et al., Axonal Guillain-Barré syndrome: a critical review, Acta Neurol Scand 2001: 103:267-277;
42. Joab Chapman et al., Antibodies to ganglioside GM₁ in patients with Alzheimer's disease, Neurosci. Lett. 86 (1988) 235-240;
43. D. Adams et al., Predictive value of anti-GM₁ ganglioside antibodies in neuromuscular diseases: a study of 180 sera; J. Neuroimmunol., 32 (1991) 223-230;
44. Nobuhiro Yuki et al., Cross-reactive antigen between nervous tissue and a bacterium elicits Guillain-Barré syndrome: Molecular mimicry between ganglioside G_{M1} and lipopolysaccharide from Penner's serotype 19 of *Campylobacter jejuni,* Biomedical Research 13 (6) 451-453 (1992) ;
45. B.Schwerer et al., Antibody cross-reactivities between gangliosides and lipopolysaccharides of *Campylobacter jejuni* serotypes associated with Guillain-Barré syndrome, J. Endotox. Res. (1995) 2, 395-403;
46. Marina Bersudsky et al., Lipopolysaccharides of a *Campylobacter coli* isolate from a patient with Guillain-Barré syndrome display ganglioside mimicry, Neuromuscular Disorders 10 (2000) 182-186;
47. Martina M. Prendergast et al., Lipopolysaccharides in the development of the Guillain-Barre syndrome and Miller Fisher syndrome forms of acute inflammatory peripheral neuropathies, J. Endotox. Res., Vol.6, No.5, 2000, 341-359;
48. Andrea Neisser et al., Serum Antibodies against Gangliosides and *Campylobacter jejuni* Lipopolysaccharides in Miller Fisher Syndrome, Infect.Immun. 65:4038-4042 (1997) ;
49. Michiaki Koga et al., Close association of IgA anti-ganglioside antibodies with antecedent *Campylobacter jejuni* infection in Guillain-Barré und Fisher's syndromes, J. Neuroimmunol. 81 (1998) 138-143;
50. Michiaki Koga et al., Subclass distribution and the secretory component of serum IgA anti-ganglioside antibodies in Guillain-Barré syndrome after *Campylobacter jejuni* enteritis, J. Neuroimmunol. 96 (1999) 245-250;
51. M. Mori et al., *Haemophilus influenzae* infection and Guillain-Barre syndrome, Brain (2000), 123, 2171-2178;
52. Yoram Nevo et al., Acute Immune Polyneuropathies: Correlations of Serum Antibodies to *Campylobacter jejuni* and *Helicobacter pylori* with Anti-GM₁ Antibodies and Clinical Patterns of Disease, J.Infect.Dis. 1997; 176(Suppl.2): S154-6;
53. T. McAlarney et al., Specifity and Cross-Reactivity of Anti-Galactocerebroside Antibodies, Immunol.Invest., 24(4), 595-606 (1995);
54. C.W.Ang et al., Guillain-Barre Syndrome- and Miller Fisher Syndrome-Associated *Campylobacter jejuni* Lipopolysaccharides Induce Anti-GM₁ and Anti-GQ_{1b} Antibodies in Rabbits, Infect.Immun. Vol.69, No.4:2462-2469 (2001) ;
55. Steven Petratos et al., Antibodies against peripheral myelin glycolipids in people with HIV infection, Immunol.Cell Biol (1998), 76, 535-541;
56. M.Gisslén et al., Cerebrospinal Fluid Antibodies Directed against Neuron-Associated Gangliosides in HIV-1 Infection, Infection 28, 2000, No.3:143-148;
57. C.Müller et al., Characterization of Autoantibodies to Natural Killer Cells in HIV-Infected Patients, Scand.J.Immunol. 43, 583-592, 1996;
58. Grazyna Adler et al., Small cell lung cancer is not associated with the presence of anti-fucosyl-GM1 ganglioside autoantibodies reactive in immunoenzymatic test, Lung Cancer 34 (2001) 383-385;
59. Aleksandra Lewartowska et al., Ganglioside reactive antibodies of IgG and IgM class in sera of patients with differentiated thyroid cancer, Immunol.Lett. 80 (2002) 129-132;
60. Manousos M. Konstandoulakis et al., Autoantibodies in the Serum of Patients with Gastric Cancer: Their Prognostic Importance; Hybridoma, Vol.17, No.5, 1998, 431-435;
61. Olle Nilsson, Carbohydrate antigens in human lung carcinomas, APMIS Suppl. 27, vol.100, 149-164, 1992;
62. Michael Weller et al., Ganglioside antibodies: a lack of diagnostic specifity and clinical utility? J Neurol (1992) 239:455-459;
63. P.A. McCombe et al., Results of testing for anti-GM1 antibodies, J. Clin. Neurosci. (2000) 7(3), 209-212;
64. Einar Bech et al., ELISA-Type Titertray Assay of IgM Anti-GM1 Autoantibodies, Clin.Chem. 40/7, 1331-1334 (1994);
65. Alan Pestronk, MD et al., Multifocal motor neuropathy: Serum IgM anti-GM1 ganglioside antibodies in most patients detected using covalent linkage of GM1 to ELISA plates, Neurology 1997; 49:1289-1292;
66. Mepur H. Ravindranath et al., Factors affecting the fine specifity and sensitivity of serum antiganglioside antibodies in ELISA, J.Immunol.Methods 169 (1994) 257-272;
67. Armin Alaedini et al., Detection of anti-GM1 Ganglioside Antibodies in Patients with Neuropathy by a Novel Latex Agglutination Assay, J.Immunoassay, 21(4), 377-386 (2000);
68. Armin Alaedini et al., Ganglioside Agglutination Immunoassay for Rapid Detection of Autoantibodies in Immune-Mediated Neuropathy, J.Clin.Lab.Anal. 15:96-99, 2001;

## Patentansprüche

1. *In vitro* Verfahren zur Früherkennung von Neoplasmen bei einem Patienten und zur Abschätzung seiner Gefährdung durch eine Ausbildung und/oder Ausbreitung maligner Neoplasmen, **dadurch gekennzeichnet, dass** man in einer Körperflüssigkeit des Patienten die Anwesenheit und Menge von an Asialo-G_{M1} (AG_{M1}) bindenden Antikörpern (anti-AG_{M1}-Antikörper) vom IgG- und/oder IgA-Typ bestimmt und deren Anwesenheit und/oder gegenüber Normalpersonen signifikant erhöhte Mengen mit dem Vorliegen eines Neoplasmas und/oder einer Gefährdung des Patienten durch maligne Neoplasmen korreliert.

2. Verfahren nach Anspruch 1, **dadurch gekennzeichnet, dass** man die Antikörperbestimmung mit Hilfe eines Liganden-Bindungsassays (Immunoassays) vom Sandwichtyp oder vom kompetitiven Typ durchführt.

3. Verfahren nach Anspruch 2, **dadurch gekennzeichnet, dass** man man die Antikörperbestimmung mit Hilfe eines Ligandenbindungs-Assays unter Verwendung eines mit einem Chemilumineszenz-Markierungsmolekül markierten Reagens durchführt.

4. Verfahren nach einem der vorausgehenden Ansprüche, **dadurch gekennzeichnet, dass** man den Nachweis der Antikörper mit einem Nachweis von Neoplasmen, die einer Krebserkrankung eines inneren Organs oder einer metastasierenden Krebserkrankung zuzuordnen sind, korreliert.

5. Verfahren nach Anspruch 4, **dadurch gekennzeichnet, dass** die Krebserkrankung ein Colon-Karzinom, ein Mamma-Karzinom, ein Ovarial-Karzinom, ein Pankreas-Karzinom, ein Ösophagus-Karzinom, ein Gallen-Karzinom, ein Leber-Karzinom, ein C-Zell-Karzinom, ein Schilddrüsen-Karzinom, ein Prostata-Karzinom, ein Lungen-Karzinom, ein Appendix-Karzinom, ein Blasen-Karzinom, ein Cardia-Karzinom, ein Karzinom des distalen Verophagus, ein Mundboden-Karzinom, ein Nieren-Karzinom oder ein Oberbauchkarzinom ist.

6. Verfahren nach einem der Ansprüche 1 bis 3, **dadurch gekennzeichnet, dass** der Patient ein Patient mit einer entzündlichen Darmerkrankung ist und der Nachweis von signifikant erhöhten Mengen von an AG_{M1} bindenden Antikörpern mit einem erhöhten Darmkrebs-Risiko bei diesem Patienten korreliert wird.

7. Verfahren nach einem der Ansprüche 1 bis 6, **dadurch gekennzeichnet, dass** man vor der Messung und/oder bei der Auswertung der erhaltenen Meßergebnisse bei dem Patienten Neuropathien, die von erhöhten Titern von an AG_{M1} bindenden Antikörpern begleitet sind, ausschließt.

8. *In vitro* Verwendung von an AG_{M1} bindenden Autoantikörpern vom IgG- und/oder IgA-Typ als universelle Marker für die Früherkennung und Prognose von malignen neoplastischen Erkrankungen und für die Abschätzung des Risikos eines Patienten, dass sich bei ihm eine maligne neoplastische Erkrankung entwickelt.

## Claims

1. *In vitro* method for the early diagnosis of neoplasms in a patient and for estimating his risk from the formation and/or spread of malignant neoplasms, **characterized in that** the presence and amount of antibodies of the IgG and/or IgA type which bind to asialo-G_{M1} (AG_{M1}) (anti-AG_{M1-}antibodies) are determined in a body fluid of the patient and their presence and/or significantly increased amounts compared with normal persons are correlated with the presence of a neoplasm and/or with a risk to the patient from malignant neoplasms.

2. Method according to Claim 1, **characterized in that** the antibody determination is carried out with the aid of a ligand binding assay (immunoassay) of the sandwich type or the competitive type.

3. Method according to Claim 2, **characterized in that** the antibody determination is carried out with the aid of a ligand binding assay using a reagent labeled with a chemiluminescence labeling molecule.

4. Method according to any of the preceding Claims, **characterized in that** the detection of the antibodies is correlated with a detection of neoplasms to be assigned to a cancer disease of an internal organ or to a metastatic cancer disease.

5. Method according to Claim 4, **characterized in that** the cancer disease is a carcinoma of the colon, a carcinoma of the breast, an ovarian carcinoma, a carcinoma of the stomach, a carcinoma of the pancreas, a carcinoma of the oesophagus, a carcinoma of the gall bladder, a carcinoma of the liver, a C cell carcinoma, a carcinoma of the thyroid, a carcinoma of the prostate, a carcinoma of the lung, a carcinoma of the appendix, a carcinoma of the bladder, a cardia carcinoma, a carcinoma of the distal verophagus, a carcinoma of the floor of the mouth, a carcinoma of the kidney or an epigastric carcinoma.

6. Method according to any of Claims 1 to 3, **characterized in that** the patient is a patient having an inflammatory bowel disease, and the detection of significantly increased amounts of antibodies binding to AG_{M1} is correlated with an increased risk of intestinal cancer in this patient.

7. Method according to any of Claims 1 to 6, **characterized in that**, before the measurement and/or in the evaluation of the measured results obtained, neuropathies which are accompanied by increased titres of antibodies binding to AG_{M1} are ruled out in the patient.

8. *In vitro* use of autoantibodies of the IgG and/or IgA type which bind to AG_{M1} as universal markers for the early diagnosis and prognosis of malignant neoplastic diseases and for the estimation of the risk to a patient that a malignant neoplastic disease will develop in said patient.

## Revendications

1. Procédé *in vitro* de dépistage précoce de néoplasmes chez un patient, et d'appréciation de sa mise en danger par une formation et/ou une propagation de néoplasmes malins, **caractérisé en ce que**, dans un fluide biologique d'un patient, on détermine la présence et la quantité d'anticorps se liant à asialo-G_{M1} (anticorps anti-AG_{M1}) de type IgG et/ou IgA, et on établit une corrélation entre leur présence et/ou leurs quantités accrues de manière significative par rapport à des personnes normales, et la présence d'un néoplasme et/ou d'une mise en danger du patient par des néoplasmes malins.

2. Procédé selon la revendication 1, **caractérisé en ce que** l'on effectue la détermination des anticorps à l'aide d'un test de liaison de ligand de type sandwich ou de type compétitif.

3. Procédé selon la revendication 2, **caractérisé en ce que** l'on effectue la détermination des anticorps à l'aide d'un test de liaison de ligand en utilisant un réactif marqué avec une molécule de marquage par chimioluminescence.

4. Procédé selon l'une des revendications précédentes, **caractérisé en ce que** l'on établit une corrélation entre la détection des anticorps et une indication de néoplasmes qui sont associés à une maladie cancéreuse d'un organe interne ou à une maladie cancéreuse à métastases.

5. Procédé selon la revendication 4, **caractérisé en ce que** la maladie cancéreuse est un cancer du colon, un cancer du sein, un cancer de l'ovaire, un cancer du pancréas, un cancer de l'oesophage, un cancer de la vésicule biliaire, un cancer du foie, un cancer des cellules C, un cancer de la thyroïde, un cancer de la prostate, un cancer du poumon, un cancer de l'appendice, un cancer de la vessie, un cancer du coeur, un cancer du verophage distal, un cancer du plancher de la bouche, un cancer du rein ou un cancer de l'épigastre.

6. Procédé selon l'une des revendications 1 à 3, **caractérisé en ce que** le patient est un patient présentant une maladie inflammatoire de l'intestin, et on établit une corrélation entre la détection de quantités significativement accrues d'anticorps se liant à AG_{M1} et un risque accru de cancer de l'intestin chez ce patient.

7. Procédé selon l'une des revendications 1 à 6, **caractérisé en ce que** l'on exclut avant la mesure et/ou lors de l'évaluation des résultats de mesure obtenus chez le patient, des neuropathies qui s'accompagnent de titres accrus d'anticorps se liant à AG_{M1}.

8. Utilisation *in vitro* d'anticorps se liant à AG_{M1}, de type IgG et/ou IgA comme marqueur universel pour le dépistage précoce et le pronostic de maladies néoplasiques malignes, et pour l'appréciation du risque pour un patient de développer une maladie néoplasique maligne.
